(19) Europäisches Patentamt
European Patent Office
Office européen des brevets

(11) **EP 2 900 183 B1**

(12) **EUROPÄISCHE PATENTSCHRIFT**

(45) Veröffentlichungstag und Bekanntmachung des Hinweises auf die Patenterteilung:
**07.06.2017   Patentblatt 2017/23**

(21) Anmeldenummer: **13756866.3**

(22) Anmeldetag: **28.08.2013**

(51) Int Cl.:
**A61F 9/00** *(2006.01)*        **B65D 65/38** *(2006.01)*
**B65D 47/20** *(2006.01)*      **B05B 11/00** *(2006.01)*

(86) Internationale Anmeldenummer:
**PCT/EP2013/067847**

(87) Internationale Veröffentlichungsnummer:
**WO 2014/048668 (03.04.2014 Gazette 2014/14)**

(54) **FLÜSSIGKEITSSPENDER**

LIQUID DISPENSER

DISTRIBUTEUR DE LIQUIDES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **25.09.2012   DE 102012217338**

(43) Veröffentlichungstag der Anmeldung:
**05.08.2015   Patentblatt 2015/32**

(73) Patentinhaber: **Aptar Radolfzell GmbH 78315 Radolfzell (DE)**

(72) Erfinder: **RITSCHE, Stefan 78253 Eigeltingen (DE)**

(74) Vertreter: **Patentanwaltskanzlei Cartagena Partnerschaftsgesellschaft Klement, Eberle mbB Urbanstraße 53 70182 Stuttgart (DE)**

(56) Entgegenhaltungen:
**WO-A1-2012/084557      WO-A2-2007/109166 DE-B4- 10 201 110**

EP 2 900 183 B1

## Beschreibung

### Anwendungsgebiet und Stand der Technik

Die Erfindung betrifft einen Flüssigkeitsspender zum Austrag pharmazeutischer Flüssigkeiten mit einem Gehäuse, einer Auslassöffnung und einem der Auslassöffnung zugeordneten Auslassventil. Dabei umfasst das Auslassventil eines solchen gattungsgemäßen Flüssigkeitsspenders als Hauptkomponenten eine der Auslassöffnung vorgeschaltete oder mit der Auslassöffnung identische Ventilöffnung und einen Ventilstift, wobei der Ventilstift in einer Schließlage des Auslassventils die Ventilöffnung verschließt und in einer Öffnungslage des Auslassventils die Ventilöffnung freigibt. Weiterhin umfasst das Auslassventil einen Ventilkörper, der entweder den Ventilstift oder die Ventilöffnung aufweist und der partiell gegenüber dem Gehäuse des Flüssigkeitsspenders verlagerbar ist, um hierdurch die Überführung des Auslassventils zwischen der genannten Schließlage und der genannten Öffnungslage zu ermöglichen. Teil des Ventils ist auch eine der Ventilöffnung vorgelagerte Druckkammer, die zu einer Seite hin durch ein Ventilteller des Ventilkörpers begrenzt ist, wobei der Ventilkörper mittels Druckbeaufschlagung durch Flüssigkeit einer Druckkammer abschnittsweise gegenüber dem Gehäuse verlagerbar ist, so dass hierdurch der Ventilstift und die Ventilöffnung gegeneinander verlagert werden können.

Insbesondere weist ein gattungsgemäßer Flüssigkeitsspender eine Druckkammer auf, bei der ein besonderes Verhältnis zwischen dem Volumen $V_{DK}$ der Druckkammer und der ventilkörperseitigen der Druckkammer zugewandten Druckkammeroberfläche Druckkammeroberfläche besteht, nämlich ein Verhältnis, demzufolge der Quotient aus Druckkammervolumen durch Druckkammeroberfläche im Intervall zwischen 0,1 mm und 2,4 mm liegt.

Gattungsgemäße Flüssigkeitsspender dienen vor allem dem Austrag pharmazeutischer Flüssigkeiten und sind insbesondere vorzugsweise für eine Abgabe der Flüssigkeit in Form einzelner Tropfen vorgesehen. Das Auslassventil solcher Spender öffnet und erlaubt somit die Abgabe von Flüssigkeit, wenn ein ausreichend großer Flüssigkeitsdruck in der Druckkammer gegeben ist.

Ein Flüssigkeitsspender, dessen Auslassventil zumindest in etwa den genannten Parametern entspricht, ist aus der DE 10 2009 006 428 A1 bekannt. Bei der dort vorgeschlagenen Gestaltung wie auch anderen gattungsgemäßen Gestaltungen wird in der genannten Art zum Zwecke des Flüssigkeitsaustrags innerhalb eines Flüssigkeitsreservoirs des Spenders ein Druck erzeugt, beispielsweise durch unmittelbare volumetrische Verkleinerung des Flüssigkeitsspeichers. Dieser Druck stellt sich dann ebenfalls in der Druckkammer ein und wirkt dort auf den Ventilteller. Da dieser zumindest partiell gegenüber dem Gehäuse verlagerbar ist, findet eine solche Verlagerung unter dem Einfluss dieses Drucks statt, durch die der Ventilstift und die Ventilöffnung voneinander beabstandet werden, so dass Flüssigkeit durch die Ventilöffnung hindurch nach außen treten kann. Im Fall der Ausgestaltung gemäß der DE 10 2009 006 428 A1 ist der Ventilstift mit dem Ventilteller verbunden, während die Ventilöffnung Teil des Gehäuses ist. Ebenso kann jedoch auch ein zum Gehäuse feststehender Ventilstift Verwendung finden, demgegenüber eine am Ventilkörper vorgesehene Ventilöffnung verlagert wird. Auch dies ist grundsätzlich aus dem Stand der Technik bekannt.

Der Zweck des Ventiltellers liegt bei gattungsgemäßen Spendern insbesondere darin, eine vergleichsweise große Oberfläche zur Verfügung zu stellen, so dass bereits bei einem vergleichsweise geringen Überdruck in der Druckkammer die gewünschte Verlagerung stattfindet. Dies spielt beispielsweise im Falle von Tropfenspendern eine erhebliche Rolle, bei denen gewünscht ist, dass die Flüssigkeit mit nur geringem Überdruck durch die Ventilöffnung nach außen tritt.

Die vergleichsweise große, der Ventilkammer zugewandte Oberfläche des Ventilkörpers im Bereich des Ventiltellers führt jedoch zu einem Problem. So wurde festgestellt, dass die übliche Materialwahl für den Ventilteller, nämlich die Verwendung thermoplastischer Elastomere (TPE), zu einer Verunreinigung der Flüssigkeit führen kann. Gerade im Bereich der Druckkammer, in welcher jener Teil der Flüssigkeit gelagert ist, der im Zuge eines der nächsten Auftragsvorgänge ausgetragen wird, führen die vergleichsweise große Flüssigkeitskontaktfläche am Ventilkörper und ein vergleichsweise geringes Volumen der Druckkammer dazu, dass in der Druckkammer die Verunreinigung am höchsten ist. Die Verunreinigung erklärt sich insbesondere aus Additiven, die bei der TPE-Herstellung Verwendung finden, um spezifische Eigenschaften zu verursachen, wie beispielsweise das gewünschte Maß an gegebenenfalls erforderlicher Verformbarkeit. Diese Additive neigen dazu, auf die Dauer in geringen Mengen aus ihrem Trägermaterial zu entweichen und dadurch die genannte Verunreinigung zu bewirken. Da zwischen Austragvorgängen eines gattungsgemäßen Flüssigkeitsspenders mitunter lange Zeiträume liegen können, kann es im Bereich der Druckkammer und somit im zum Austrag anstehenden Teil der Flüssigkeit zu einem inakzeptablen Maß an Verunreinigungen kommen.

Aus dem Stand der Technik, nämlich der EP 0791397 B1, ist es im Zusammenhang mit Austragvorrichtungen für Medien bekannt, Kunststoffbauteile einer Austragvorrichtung unter Verwendung von mit Metallocenen herzustellen.

WO 2012/084557 A1 offenbart einen Flüssigkeitspender und eine entsprechende Verwendung gemäß den Oberbegriffen der Ansprüche 1 und 11. DE 102 01 110 A1 offenbart Behälter zur Aufnahme von medizinischen oder pharmazeutischen Lösungen aus einem Kunststoffmaterial, das Copolymere aufweist, die unter Verwendung von Cycloolefinen hergestellt werden.

## Aufgabe und Lösung

Aufgabe der Erfindung ist es, einen gattungsgemäßen Flüssigkeitsspender dahingehend weiterzubilden, dass das Problem der verstärkten Verunreinigungen im Bereich der Druckkammer vermindert wird.

Erfindungsgemäß wird hierzu gemäß einem ersten Aspekt vorgeschlagen, dass zumindest ein Teilabschnitt des Ventiltellers, der mit der Flüssigkeit in der Druckkammer in Kontakt ist, aus einem Ventiltellermaterial besteht, bei dem es sich um einen Kunststoff handelt, welcher unter Verwendung von Metallocenen als Katalysatoren hergestellt ist.

Gemäß einem zweiten Aspekt wird vorgeschlagen, dass ein Kunststoff, welcher unter Verwendung von Metallocenen als Katalysatoren hergestellt ist, zumindest für einen Teilabschnitt des Ventiltellers verwendet wird, der mit der Flüssigkeit in der Druckkammer in Kontakt ist.

Die erfindungsgemäße Gestaltung eines gattungsgemäßen Flüssigkeitsspenders sieht demnach vor, dass jenes Bauteil, welches einen erheblichen Anteil der Wandungsfläche der Druckkammer bildet, aus einem Kunststoff hergestellt ist, der mit Metallocen-Katalysatoren hergestellt wurde. Im Weiteren wird ein solcher Kunststoff als Metallocen-Kunststoff bezeichnet. Der erfindungsgemäßen Gestaltung liegt die Erkenntnis zugrunde, dass die Verunreinigung der Flüssigkeit durch Additive des Kunststoffs im Bereich des gattungsgemäß ausgestalteten Druckraums eine sehr viel größere nachteilige Wirkung als an anderen flüssigkeitsführenden Bereichen des Flüssigkeitsspenders entfaltet, da hier zum einen ein sehr ungünstiges Verhältnis zwischen der Größe der Wandungsflächen und dem Flüssigkeitsvolumen besteht und da zum anderen jene Flüssigkeit, die in der Druckkammer angeordnet ist, bei der jeweils nächsten oder einer der jeweils nächsten Austragbetätigungen ausgetragen wird. Eine Vermengung mit weniger verunreinigten Teilen der Flüssigkeit des Spenders, die zu einer Senkung der spezifischen Verunreinigungsmenge führen könnte, findet üblicherweise nicht mehr statt.

Es hat sich gezeigt, dass in hervorragender Art und Weise die Verunreinigung verhindert oder vermindert werden kann, wenn zumindest Teilabschnitte des Ventiltellers oder aber der gesamte Ventilteller bzw. seine Druckkammeroberfläche aus dem genannten Material hergestellt sind.

In Hinblick auf das Material wird auf die EP 0791397 B1 verwiesen, deren Offenbarungsteile über unter Verwendung von Metallocenen hergestellte Kunststoffe und deren Eigenschaften durch ausdrückliche Bezugnahme vollumfänglich zum Teil der Offenbarung dieses Dokuments gemacht werden.

Im Übrigen ist in Hinblick auf die erfindungsgemäß zu verwendenden Materialien weiterhin folgendes ergänzend festzustellen.

Bei dem erfindungsgemäß verwendeten Kunststoff handelt es sich um ein Cyclo-Olefin-Copolymer oder um eine Kombination aus einem Cyclo-Olefin-Copolymer und einem Polypropylen.

Wie bereits erwähnt, erfolgt die Herstellung des erfindungsgemäß verwendeten Kunststoffs mittels Metallocenen als Katalysatoren. Geeignete Metallocen-Katalysatoren können aus der Gruppe umfassend Alkalimetallocene, Erdalkalimetallocene, Metallocene mit Metallatomen aus der vierten bis zwölften Nebengruppe des chemischen Periodensystems und Kombinationen davon ausgewählt sein.

Insbesondere können geeignete Metallocen-Katalysatoren ausgewählt sein aus der Gruppe umfassend Titanocen, Zirkonocen, Vanadocen, Chromocen, Manganocen, Ferrocen, Cobaltocen, Nickelocen, Zinkocen, Niobocen, Molybdocen, Ruthenocen, Rhodocen, Tantalocen, Wolframocen, Rhenocen, Osmocen, Iridocen, Platinocen und Kombinationen davon.

Die Metallocene können insbesondere als Co-Katalysatoren vorgesehen sein.

Die Metallocene sind vorzugsweise in einem Gewichtsanteil von weniger als 5/10000000, insbesondere weniger als 1/10000000, im Kunststoff enthalten.

Die Metallocen-Katalysatoren können im Bauteil verbleiben oder nach dessen Herstellung auch wieder von diesem abgetrennt werden.

Üblicherweise initiieren und insbesondere beschleunigen die Metallocen-Katalysatoren den für die Herstellung des Kunststoffs erforderlichen Polymerisationsvorgang, wobei sie insbesondere auch für eine sehr enge Molmassenverteilung des entstehenden Polymers bzw. Kunststoffs verantwortlich sind.

In einer vorteilhaften Ausführungsform enthält der erfindungsgemäß verwendete Kunststoff, gegebenenfalls abgesehen von etwaigen unter medizinischen bzw. pharmazeutischen Gesichtspunkten vernachlässigbaren Metallocenspuren, keine weiteren Additive. Hierdurch lässt die Gefahr von Verunreinigungen zusätzlich reduzieren.

Das genannte Intervall, in welchem sich der Quotient von Druckkammervolumen zu Druckkammeroberfläche erfindungsgemäß befindet, entspricht näherungsweise der Spaltbreite innerhalb der Druckkammer. Dieser Spalt weist somit vorzugsweise in etwa eine Breite zwischen 0,1 mm und 2,4 mm auf. Je kleiner das Volumen ist, desto zweckmäßiger ist die erfindungsgemäße Ausgestaltung des Ventiltellers. Insbesondere ein Quotient, der zwischen 0,2 mm und 1,0 mm liegt, hat sich in der Praxis als besonders vorteilhaft herausgestellt.

Der obere Grenzwert von 2,4 mm ist dabei dadurch bedingt, dass durch eine Spaltbreite in diesem Bereich oder eine geringere Spaltbreite erreicht wird, dass nur eine geringe Menge Luft in der Druckkammer verbleibt, die den Austragvorgang negativ beeinflussen könnte. Durch eine mittlere Spaltbreite kleiner gleich 1,0 mm kommt es dazu, dass auch bei einer Lagerung des Spenders in einer Ausrichtung, bei der die Druckkammer oberhalb des damit verbundenen Flüssigkeitsreservoirs angeordnet ist, die Flüssigkeit aus der Druckkammer nicht oder in geringem Maße zurück in das Flüssigkeitsreservoir strömt. Somit dringt keine Luft oder eine nur geringe Luftmenge in einer solchen Ausrichtung in die Druckkammer ein, was wiederum einer verbesserten Austragcharakteristik dienlich ist. Das verminderte Ausströmen von Flüssigkeit aus der Druckkammer in das Flüssigkeitsreservoir ist vermutlich auf einen Kapillareffekt in der Druckkammer zurückzuführen.

In absoluten Zahlen wird es als besonders vorteilhaft angesehen, wenn das Druckkammervolumen zwischen 20 mm$^3$ und 100 mm$^3$ beträgt, insbesondere zwischen 30 mm$^3$ und 50 mm$^3$.

Hierbei ist zu beachten, dass als Druckkammervolumen jenes Volumen angesehen wird, welches im Ventilbereich bestimmungsgemäß von Flüssigkeit erreicht wird und welches sich dabei von einem Druckkammereinlass bis zur durch den Ventilstift verschlossenen Ventilöffnung erstreckt. Dabei ist der Druckkammereinlass jene Stelle des Flüssigkeitspfades von einem Flüssigkeitsspeicher des Spenders bis zur Druckkammer, an welchem die Querschnittsfläche des Flüssigkeitspfades ihr Minimum aufweist.

Als besonders vorteilhafte Werte für die Größe der Druckkammeroberfläche des Ventilkörpers, also jene Fläche, die zumindest zum Teil durch Metallocen-Kunststoffe gebildet wird, wird eine Größe zwischen 100 mm$^2$ und 300 mm$^2$ angesehen. Dabei wird als Druckkammeroberfläche die Gesamtheit jener Oberflächen angesehen, die sich am im Zuge des Öffnens und Schließens bewegten Teil des Ventilkörpers und ggf. dem ihm zugeordneten Ventilstift befindet und die bei geschlossenem Auslassventil die Druckkammer unmittelbar begrenzt.

Wie eingangs bereits erwähnt, sind grundsätzlich zwei unterschiedliche Gestaltungen hinsichtlich der Anordnung des Ventiltellers denkbar. Dieser kann einerseits den Ventilstift tragen und diesen somit gegenüber der gehäusefesten Ventilöffnung verlagerbar machen. Andererseits kann auch die Ventilöffnung gemeinsam mit dem Ventilteller verlagert werden, wobei in diesem Falle der Ventilstift gehäusefest angeordnet ist. Die Verwendung des Metallocen-Kunststoffs muss nicht zwingend auf den Ventilteller und ggf. zusätzlich den Ventilstift beschränkt sein. Insbesondere von Vorteil ist es, wenn zumindest zum Teil auch weitere gegenüber dem Außengehäuse des Flüssigkeitsspenders unbewegte Oberflächen, die die Druckkammer begrenzen, aus einem derartigen Kunststoff gefertigt oder mit einer Schicht aus solchem Kunststoff ausgestaltet sind. Vorzugsweise bestehen mindestens 30% der die Druckkammer begrenzenden Flächen aus Metallocen-Kunststoff.

Eine vollständige oder überwiegende Ausgestaltung des Flüssigkeitsspenders aus Metallocen-Kunststoff ist jedoch zumindest vorzugsweise nicht vorgesehen. Insbesondere ein Außengehäuse des Flüssigkeitsspenders und vorzugsweise auch jene Wandungen, die den Flüssigkeitsspeicher des Spenders begrenzen, sind vorzugsweise nicht aus dem vergleichsweise aufwändig herzustellenden Metallocen-Kunststoff hergestellt.

Der Ventilkörper ist zum Zwecke der Ermöglichung der Verlagerbarkeit der Ventilöffnung oder des Ventilstifts gegenüber dem Gehäuse in sich verformbar ausgebildet und in einem außenseitigen Festlegungsbereich am Gehäuse festgelegt. Dabei kann die Verformbarkeit alternativ zueinander in einem Bereich gegeben sein, der aus dem Metallocen-Kunststoff besteht, oder aber in einem Bereich, der aus einem hiervon abweichenden Kunststoff gebildet besteht.

Um die gewünschte Verformbarkeit in ausreichendem Maße zu erreichen, hat es sich als vorteilhaft herausgestellt, wenn im Bereich des Ventiltellers ein umlaufender Verformungsbereich vorgesehen ist in welchem der Ventilteller dünnwandiger als im radial innenseitig und außenseitig angrenzenden Bereichen ausgebildet ist. Insbesondere ist der vorzugsweise sickenartige ausgebildete umlaufende Bereich an seiner dünnsten Stelle maximal 60% so dick wie die mit maximaler Dicke ausgebildeten Bereiche innenseitig und außenseitig der Sicke.

Grundsätzlich ist es möglich, durch Elastizität des Ventilbauteils, entweder die Elastizität des Metallocen-Kunststoffs oder die Elastizität eines anderen Teils des den Ventiltellers abschnittsweise bildenden anderweitigen Kunststoffs eine Rückstellkraft des Auslassventils in seine Schließlage zu bewirken. Vorteilhaft ist es allerdings, wenn zur Herstellung der Rückstellkraft eine separate Ventilfeder vorgesehen ist, mittels derer das Auslassventil in Richtung seiner Schließlage kraftbeaufschlagt ist. Die Ventilfeder ist demnach so angeordnet, dass sie eine Kraftbeaufschlagung des Ventilstiftes in Richtung der Ventilöffnung bzw. der Ventilöffnung in Richtung des Ventilstiftes bewirkt. Durch die Verwendung einer solchen separaten Ventilfeder muss bei der Materialauswahl des Ventilkörpers keine Berücksichtigung der gewünschten Schließkraft erfolgen. Es reicht aus, wenn der Ventilkörper als Ganzes beweglich ist oder Abschnitte desselben ausreichend verformbar sind.

Um zu vermeiden, dass sich die genannte Ventilfeder an den in hohem Maße verformbaren Bereichen des Ventilkörpers abstützt, ist es von Vorteil, wenn der Ventilkörper einen Stützkörper aufweist, der gemeinsam mit dem Ventilstift bzw. der Ventilöffnung verlagerbar ist und der aus einem gegenüber dem Material oder den Materialien des Ventiltellers steiferen Kunststoff, beispielsweise Polyethylen und/oder Polypropylen, besteht. Hierdurch wird eine verbesserte Krafteinleitung der Federkraft der Ventilfeder in den Ventilkörper erzielt. Der Metallocen-Kunststoff kann dann in der Art eines Überzugs über dem Stützkörper vorgesehen sein.

Ein erfindungsgemäßer Flüssigkeitsspender dient insbesondere der Abgabe von pharmazeutischen Flüssigkeiten. Er ist daher vorzugsweise mit einer solchen Flüssigkeit befüllt, insbesondere mit einer ophtalmischen Flüssigkeiten. Insbesondere handelt es um pharmazeutische Flüssigkeiten zur Behandlung des erhöhten Augeninnendrucks (Glaukombehandlung), zur Behandlung des trockenen Auges und zur Behandlung von Allergien und Entzündungen. Diese enthalten als Bestandteile vorzugsweise Wirkstoffe aus der Gruppe umfassend Alpha-2 Agonisten, z.B. Brimonidin, Prostaglandinanaloga (Tafluprost, Latanoprost, Bimatoprost, Travoprost), Beta-Blocker, z. B. Timolol, und Carboanhydrasehemmer, z. B. Dorzolamid beziehungsweise Hyaluronsäureverbindungen, Filmbildner, z.B. Methylcelluloseverbindungen und Cyclosporin beziehungsweise Antihistaminika, z.B. Olopatadin und Levocabastin, Steroide, z.B. Loteprdnol und Dexamethason, und NSAID, z. B. Keterolac, sowie Kombinationen davon.

## Kurzbeschreibung der Zeichnungen

Weitere Aspekte und Vorteile der Erfindung ergeben sich aus der nachfolgenden Beschreibung bevorzugter Ausführungsformen der Erfindung, die anhand der Figuren erläutert sind. Dabei zeigen:

Fig. 1 bis 3      erfindungsgemäße Flüssigkeitsspender bzw. deren Austragköpfe mit einem gegenüber einem Außengehäuse verlagerbaren Ventilstift und

Fig. 4      eine Variante hierzu mit einer gegenüber dem Außengehäuse verlagerbaren Ventilöffnung.

## Detaillierte Beschreibung der Ausführungsformen

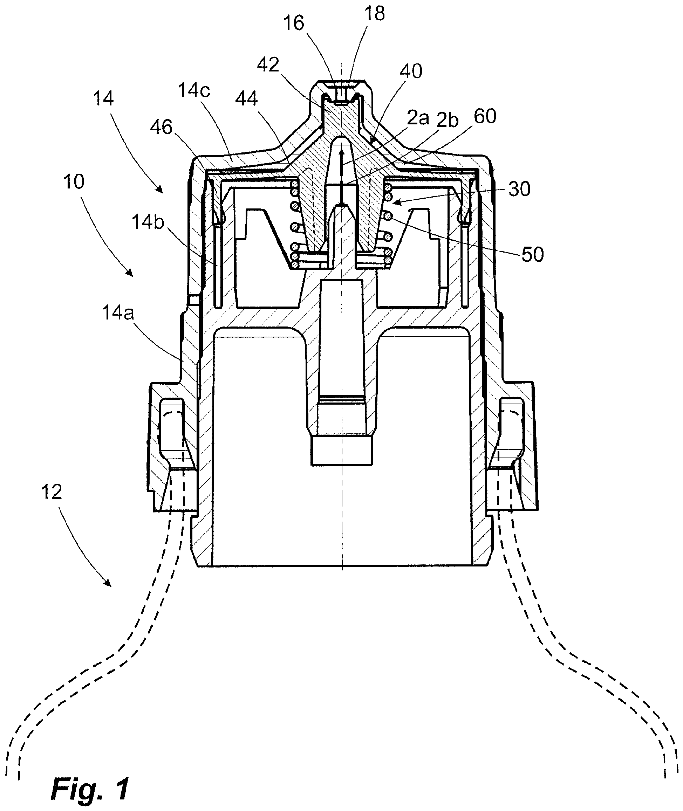

Fig. 1 zeigt einen ersten erfindungsgemäßen Flüssigkeitsspender 10, welcher zum Austrag einer pharmazeutischen Flüssigkeit in Tropfenform vorgesehen ist. Es kann sich beispielsweise um eine pharmazeutische Flüssigkeit zur Behandlung von Augen eines Patienten handeln.

Der Flüssigkeitsspender 10 umfasst einen als Flüssigkeitsspeicher dienenden Flaschenbehälter 12 sowie einen Austragkopf 14. Am Austragkopf 14 ist eine Austragöffnung 16 vorgesehen, die gleichzeitig eine Ventilöffnung 16 eines Auslassventils 30 darstellt und die ein Außengehäuse 14a des Austragkopfes 14 durchbricht. Das Auslassventil 30 ist als druckabhängig öffnendes Auslassventil 30 ausgestaltet.

Hierfür ist bezogen auf die Darstellung der Fig. 1 unterhalb der Ventilöffnung 16 ein Ventilkörper 40 angeordnet, der über einen gegenüber dem Außengehäuse 14a beweglichen Ventilstift 42 verfügt, der derart am Ventilkörper 40 angeordnet ist, dass er in einer Schließlage des Auslassventils 30 die Ventilöffnung 16 verschließen kann. Weiterhin verfügt der Ventilkörper 40 über einen Ventilteller 44, der dünnwandig ausgestaltet ist, um in nachfolgend noch erläuterter Weise eine Verformbarkeit des Ventilkörpers 40 zu gewährleisten. Außenseitig wird der Ventilköper 40 durch einen Festlegungssteg 46 begrenzt, der in einer Haltenut 14b des Gehäuses des Austragkopfes 14 befestigt ist. Mittels einer Ventilfeder 50, die sich mit Ihrem unteren Ende am Gehäuse des Austragkopfes 14 abstützt, wird der Ventilstift 42 permanent in Richtung des Pfeils 2a und somit in Richtung der Schließlage gedrückt, in der er an einem die Ventilöffnung 16 umgebenden Rand anliegt und hierdurch das Auslassventil 30 verschließt.

Das Gehäuse des Austragkopfes 14 ist in einem Stirnwandungsbereich 14c derart an die Form des Ventilkörpers 40 und dessen Ventilteller 44 angepasst, dass zwischen dieser Stirnwandung und dem Ventilkörper 44 eine Druckkammer 60 des Auslassventils 30 von sehr geringem Volumen verbleibt. Der Abstand zwischen der Innenseite der Gehäusewandung im Stirnwandungsbereich 14c und dem Ventilteller 44 des Ventilbauteils 40 beträgt etwa 0,2 mm. Jener Teil der Oberfläche des Ventilbauteils 40, der in Kontakt mit dem Inhalt der Druckkammer 60 steht und im Sinne dieses Dokuments die Druckkammeroberfläche bildet, weist eine Größe von etwa 200 mm$^2$ auf. Das Innenvolumen der Druckkammer 60 beträgt in etwa 40 mm$^3$. Die Druckkammer ist durch einen nicht näher darstellten Kanal mit dem Flüssigkeitsspeicher verbunden.

Zur Betätigung des Flüssigkeitsspenders 10 ist im Falle der Ausgestaltung der Fig. 1 ein manuelles Zusammendrücken des Flaschenbehälters 12 vorgesehen, wodurch es zu einer Druckerhöhung sowohl im Flüssigkeitsspeicher als auch in der Druckkammer 60 kommt. In Reaktion auf diese Druckerhöhung in der Druckkammer 60 wird der überwiegende Teil des Ventilkörpers 40 in Richtung des Pfeils 2b gegenüber dem Gehäuse 14a verlagert, so dass auch der Ventilstift 42 sich von dem die Ventilöffnung 16 umgebenden Rand löst und das Auslassventil 30 somit öffnet. Aufgrund der vergleichsweise großen Flächenerstreckung des Ventiltellers 44 bedarf es hierfür nur eines geringen Überdrucks. Da bereits dieser geringer Überdruck reicht, tritt die Flüssigkeit nahezu drucklos durch die Ventil- und Austragöffnung 16, was im konkreten Fall deshalb von Vorteil ist, da der Flüssigkeitsspender 10 der Fig. 1 als in Überkopflage zu verwendender Tropfenspender ausgebildet ist, bei dem vorgesehen ist, dass die austretende Flüssigkeit sich zunächst an einer Tropfenbildungsfläche 18 außenseitig der Ventil- und Austragöffnung 16 anlagert und erst bei Erreichen einer gewünschten Tropfengröße von hier löst. Ein Sprühstrahl durch zu hohen Austrittsdruck ist aufgrund dieser Zweckbestimmung nicht gewünscht.

Die Druckkammer 60 ist naturgemäß jener Teil der flüssigkeitsführenden Komponenten des Flüssigkeitsspenders 10, in welchem jene Flüssigkeit angeordnet ist, die bei dem jeweils nächsten Austragvorgang oder einem der nächsten Austragvorgänge ausgetragen wird. Da diese Flüssigkeit stets im Zuge der jeweils vorangegangenen Betätigungen in die Druckkammer 60 gelangt ist, verweilt sie hier mit unter recht lange, beispielsweise über mehrere Tage, wenn der Spender über einen solchen Zeitraum nicht benutzt wird. Da zudem in der Druckkammer 60 nur ein geringes Flüssigkeitsvolumen vorhanden ist, welches an vergleichsweise großen Wandungsflächen des aus Kunststoff hergestellten Spenders 10 über einen langen Zeitraum anliegt, ist der Flüssigkeitsspender 10 gemäß Fig. 1 dahingehend ausgebildet, dass er eine Verunreinigung dieser Flüssigkeitsmenge mit aus den Kunststoffen entweichenden Komponenten, insbesondere verschiedenen Additiven, verhindert. Zu diesem Zweck ist vorgesehen, dass der Ventilkörper 40 der Ausgestaltung der Fig. 1 als Ganzes aus einem Kunststoff besteht, der unter Verwendung von Metallocenen als Katalysator hergestellt wurde. Der Kunststoff enthält ein Cyclo-Olefin-Copolymer, insbesondere ein Copolymer, welches aus Ethen- und Norbornen-Monomereinheiten zusammengesetzt ist. Ein derartiges Co-polymer ist unter der Bezeichnung Topas kommerziell erhältlich. Ein solcher Kunststoff weist nur eine vergleichsweise geringe Menge von Additiven bzw. eine geringe Neigung, Additive in die Druckkammer abzugeben, auf.

Hierdurch wird erreicht, dass zumindest von Seiten des Ventilkörpers keine oder nur in geringem Maße Additive in die Flüssigkeit in der Druckkammer 60 gelangen. Auch bei langer Verweilzeit der Flüssigkeit in der Druckkammer 60 kommt es somit nicht in einem problematischen Maße zu einer Verunreinigung.

Bei einer nicht dargestellten Variante zu der Ausgestaltung der Fig. 1 kann auch die Innenseite der Stirnflächenwandung 14c, die an die Druckkammer 60 angrenzt, mit einem Überzug aus dem gleichen Kunststoff oder einem anderen unter der Verwendung von Metallocenen als Katalysator hergestellten Kunststoff überzogen sein, so dass ein noch höherer Anteil jener Oberflächen die die Druckkammer unmittelbar begrenzen, im Hinblick auf Verunreinigungen weitgehend unkritisch ist. Allerdings ist gerade im Bereich des Ventilkörpers die Verwendung solcher Kunststoffe von Vorteil, da sich die gewünschte Verformbarkeit des Ventiltellers 44 hierdurch erreichen lässt, ohne die Nachteile der vielfältigen Additive üblicherweise hier verwendeter thermoplastischer Elastomere in Kauf nehmen zu müssen.

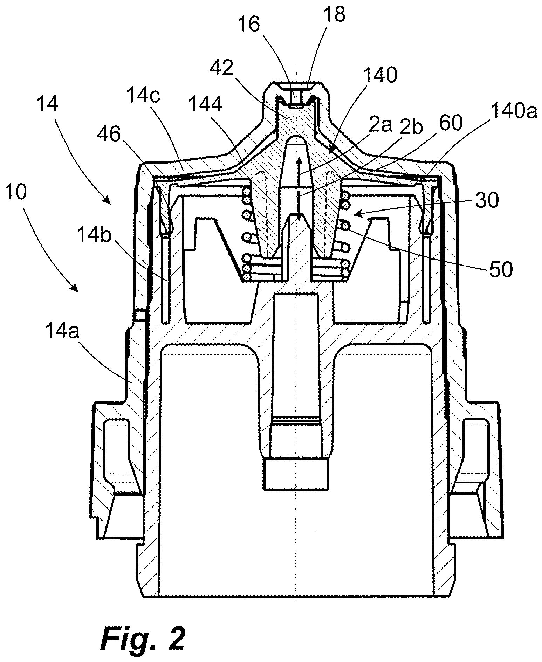

Die Ausgestaltung gemäß Fig. 2 unterscheidet sich von der Ausgestaltung gemäß Fig. 1 dadurch, dass zur Erzielung einer noch leitgängigeren Verformbarkeit des Ventilkörpers 140 in dessen Randbereich eine Sicke 140a eingebracht ist. Die im Zuge des Wechsels zwischen der Schließlage und der Öffnungslage erforderliche Verformung des Ventilkörpers 140 erfolgt somit zum überwiegenden Teil im Bereich dieser Sicke 140a. Dies ermöglicht es, bei der Auslegung des verwendeten Metalocenen-Katalysator basierten Kunststoffs für den Ventilkörper 140 die Verformbarkeit in dickwandigeren Bereichen nicht in den Vordergrund bei der Ausgestaltung des Kunststoffs stellen zu müssen. Im Übrigen entspricht die Funktionsweise bei der Ausgestaltung gemäß Fig. 2 der der gemäß Fig. 1.

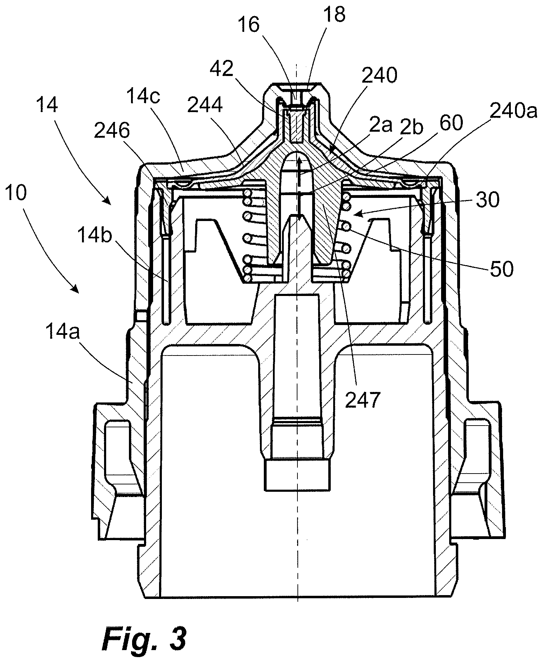

Die Ausgestaltung der Fig. 3 zeigt eine Variation, bei der der Ventilkörper 240 nochmals komplexer gestaltet ist, nämlich dadurch, dass er aus zwei verschiedenen Kunststoffen besteht, die beispielsweise durch Mehrkomponentenspritzguss zusammengefügt sein können. Während der überwiegende Teil der Druckkammeroberfläche des Ventilkörpers, die mit der Flüssigkeit in der Druckkammer 60 in unmittelbarem Kontakt steht, und insbesondere der Ventilteller 244 mitsamt Sicke 240a, wiederum aus Kunststoff besteht, der mittels Metallocen-Katalysatoren hergestellt wurde, sind sowohl der Befestigungssteg 246 als auch ein zentrisch angeordnetes Stützbauteil 247 aus einem steiferen Kunststoff hergestellt, der im Falle des Stegs 246 ein einfacheres Fügen der Bauteile während der Montage gestattet bzw. im Fall des Stützbauteils 247 eine großflächigere Krafteinleitung der Ventilfederkraft in den Ventilkörper 240 gewährleistet.

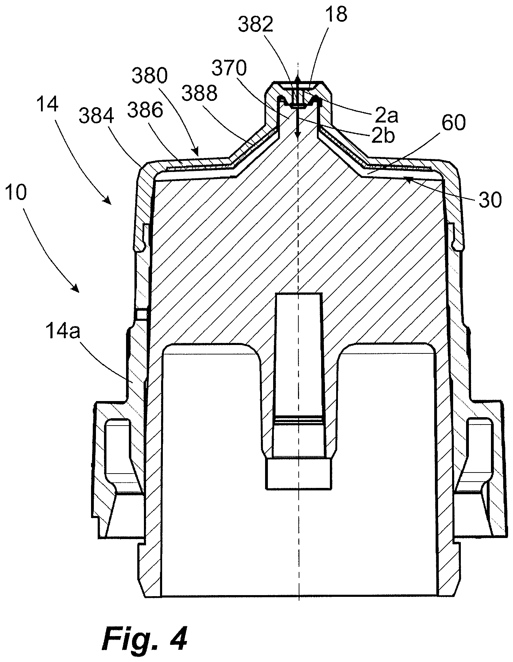

Die Ausgestaltung der Fig. 4 unterscheidet sich in erheblichem Maße von den vorangegangenen Ausführungsformen, da hier abweichend von den vorangegangenen Ausführungsformen der Ventilstift 370 ortsfest zum Gehäuse 14a des Austragkopfes 14 vorgesehen ist.

Der verlagerbare Ventilkörper 380 bildet im Falle der Ausgestaltung der Fig. 4 die Stirnfläche des Spenders, so dass die Ventilöffnung 382 im Falle einer Druckbeaufschlagung der Druckkammer 60 in Richtung des Pfeils 2a vom feststehenden Ventilstift 370 abgehoben wird. Der Ventilkörper 380 ist als Ganzes aus solchen Kunststoffen gefertigt, die ausreichend verformbar sind, um das Abheben der Ventilöffnung 382 zu gestatten. Der Ventilkörper 380 ist dabei ausreichend elastisch, um selbst die Rückstellkraft in Richtung der Schließlage zu bieten. Alternativ kann jedoch auch hier eine separate Ventilfeder vorgesehen sein. Diese würde dann von oben in Richtung des Pfeils 2b auf die Stirnfläche des Spenders wirken.

Der Ventilkörper 380 besteht aus zwei unterschiedlichen Kunststoffmaterialien. So ist eine Außenhülle 384 aus einem konventionellen Kunststoff, beispielsweise einem thermoplastischen Elastomer gefertigt, während eine innenseitig auf den Ventilteller 386 des Ventilkörpers 384 aufgebrachte Beschichtung 388 wiederum aus einem Kunststoff der bereits erwähnten Kunststoffe besteht, bei denen zur Herstellung Metallocene als Katalysatoren Verwendung finden. Damit ist wiederum die Gefahr einer Verunreinigung jener Flüssigkeitsanteile, die in der Druckkammer 60 angeordnet sind, deutlich verringert.

**Patentansprüche**

1.  Flüssigkeitsspender (10) zum Austrag pharmazeutischer Flüssigkeiten mit

    - einem Gehäuse (12, 14a),
    - einer Auslassöffnung (16) und
    - einem der Auslassöffnung (16) zugeordneten Auslassventil (30),

wobei

das Auslassventil (30) umfasst:

- eine Ventilöffnung (16; 382) und einen Ventilstift (42; 370), wobei der Ventilstift (42; 370) in einer Schließlage des Auslassventils die Ventilöffnung (16; 382) verschließt und in einer Öffnungslage des Auslassventils die Ventilöffnung freigibt,

- einen Ventilkörper (40; 140; 240; 380), der entweder den Ventilstift (42) oder die Ventilöffnung (382) aufweist und der partiell gegenüber dem Gehäuse (12, 14a) des Flüssigkeitsspenders (10) verlagerbar ist, um hierdurch die Überführung des Auslassventils (30) zwischen der Schließlage und der Öffnungslage zu ermöglichen,

- eine der Ventilöffnung (16, 382) vorgelagerte Druckkammer (60), die zu einer Seite hin durch einen Ventilteller (44; 386) des Ventilkörpers (40; 140; 240; 380) begrenzt ist, wobei der Ventilkörper (40; 140; 240; 380) mittels Druckbeaufschlagung an dem Ventilteller (44; 386) durch Flüssigkeit in der Druckkammer (60) zumindest abschnittsweise verlagerbar ist, so dass hierdurch der Ventilstift (42; 370) und die Ventilöffnung (16; 382) gegeneinander verlagert werden,

- der Ventilkörper (40; 140; 240; 380) zum Zweck der Ermöglichung der Verlagerbarkeit der Ventilöffnung (382) oder des Ventilstiftes (42) gegenüber dem Gehäuse (12, 14a) in sich verformbar ausgebildet ist und einen außenseitigen Festlegungsbereich (46, 246) aufweist, der ortsfest zum Gehäuse (12, 14a) festgelegt ist, **dadurch gekennzeichnet, dass**

- zumindest ein Teilabschnitt des Ventiltellers (44; 144; 244; 386), der mit Flüssigkeit in der Druckkammer in Kontakt ist, aus einem Ventiltellermaterial besteht, bei dem es sich um einen Kunststoff handelt, welcher unter Verwendung von Metallocenen als Katalysatoren hergestellt werden kann und es sich bei dem Kunststoff um ein Cyclo-Olefin-Copolymer oder um eine Kombination aus einem Cyclo-Olefin-Copolymer und einem Polypropylen handelt.

2. Flüssigkeitsspender (10) nach Anspruch 1, **dadurch gekennzeichnet, dass**

- die Druckkammer (60) ein Druckkammervolumen $V_{DK}$ aufweist und wobei der Ventilkörper (40; 140; 240; 380) eine zur Druckkammer hin weisende Druckkammeroberfläche $A_{DK}$ aufweist, und

- wobei der Quotient aus Druckkammervolumen $V_{DK}$ geteilt durch Druckkammeroberfläche $A_{DK}$ in folgendem Intervall liegt:

$$0,1 \text{ mm} \leq V_{DK} / A_{DK} \leq 2,4 \text{ mm}$$

3. Flüssigkeitsspender (10) nach Anspruch 2, **dadurch gekennzeichnet, dass** der Quotient aus Druckkammervolumen $V_{DK}$ und Druckkammeroberfläche $A_{DK}$ in folgendem Intervall liegt:

$$0,2 \text{ mm} \leq V_{DK} / A_{DK} \leq 1,0 \text{ mm}$$

4. Flüssigkeitsspender (10) nach Anspruch 1, 2 oder 3, **dadurch gekennzeichnet, dass** das Druckkammervolumen $V_{DK}$ zwischen 20 mm$^3$ und 100 mm$^3$ beträgt, vorzugsweise zwischen 30 mm$^3$ und 50 mm$^3$.

5. Flüssigkeitsspender (10) nach einem der Ansprüche 1 bis 4, **dadurch gekennzeichnet, dass** der Druckkammeroberfläche des Ventilkörpers (40; 140; 240; 380) zwischen 100 mm$^2$ und 300 mm$^2$ beträgt.

6. Flüssigkeitsspender (10) nach einem der vorstehenden Ansprüche, **dadurch gekennzeichnet, dass** die Ventilöffnung (16) ortsfest zum Gehäuse (12, 14a) vorgesehen ist und der Ventilkörper (40; 140; 240) den Ventilstift (42) aufweist, wobei der Ventilstift (42) durch zumindest partielle Verlagerung des Ventiltellers (44; 144; 244) gegenüber der Ventilöffnung (16) verlagerbar ist.

7. Flüssigkeitsspender (10) nach Anspruch 6, **dadurch gekennzeichnet, dass** zumindest ein Teilabschnitt des Ventiltellers (44; 144; 244) und der Ventilstift (42) als einstückiges Bauteil aus dem Kunststoff ausgebildet sind, welcher unter Verwendung von Metallocenen hergestellt ist.

8. Flüssigkeitsspender (10) nach einem der vorstehenden Ansprüche, **dadurch gekennzeichnet, dass** der Ventilteller (144; 244) mit einem umlaufenden Verformungsbereich (140a, 240a) versehen ist, in dem der Ventilteller (44; 244) dünner als in radial innenseitig und außenseitig angrenzenden Bereichen ausgebildet ist.

9. Flüssigkeitsspender (10) nach einem der vorstehenden Ansprüche, **dadurch gekennzeichnet, dass** das Auslassventil (30) über eine Ventilfeder (50) verfügt, die den Ventilstift (42) und die Ventilöffnung (16) in Richtung der Schließlage kraftbeaufschlagt.

10. Flüssigkeitsspender (10) nach einem der vorstehenden Ansprüche,
**dadurch gekennzeichnet, dass** der Ventilkörper (240) einen Stützkörper (247) aufweist, der gemeinsam mit dem Ventilteller (244) verlagerbar ist und der aus einem gegenüber dem Material des Ventiltellers steiferen Kunststoff besteht.

11. Verwendung eines Kunststoffs, welcher unter Verwendung von Metallocenen als Katalysatoren hergestellt wurde, für einen Flüssigkeitsspender (10) zum Austrag pharmazeutischer Flüssigkeiten, wobei der Flüssigkeitsspender (10) umfasst

     - ein Gehäuse (12, 14a),
     - eine Auslassöffnung (16) und
     - ein der Auslassöffnung (16) zugeordnetes Auslassventil (30),

wobei das Auslassventil (30) umfasst:

     - eine Ventilöffnung (16; 382) und einen Ventilstift (42; 370), wobei der Ventilstift (42; 370) in einer Schließlage des Auslassventils die Ventilöffnung (16; 382) verschließt und in einer Öffnungslage des Auslassventils die Ventilöffnung freigibt,
     - einen Ventilkörper (40; 140; 240; 380), der entweder den Ventilstift (42) oder die Ventilöffnung (382) aufweist und der partiell gegenüber dem Gehäuse (12, 14a) des Flüssigkeitsspenders (10) verlagerbar ist, um hierdurch die Überführung des Auslassventils (30) zwischen der Schließlage und der Öffnungslage zu ermöglichen,
     - eine der Ventilöffnung (16, 382) vorgelagerte Druckkammer (60), die zu einer Seite hin durch einen Ventilteller (44; 386) des Ventilkörpers (40; 140; 240; 380) begrenzt ist, wobei der Ventilkörper (40; 140; 240; 380) mittels Druckbeaufschlagung an dem Ventilteller (44; 386) durch Flüssigkeit in der Druckkammer (60) zumindest abschnittsweise verlagerbar ist, so dass hierdurch der Ventilstift (42; 370) und die Ventilöffnung (16; 382) gegeneinander verlagert werden,
     - der Ventilkörper (40; 140; 240; 380) zum Zweck der Ermöglichung der Verlagerbarkeit der Ventilöffnung (382) oder des Ventilstiftes (42) gegenüber dem Gehäuse (12, 14a) in sich verformbar ausgebildet ist und einen außenseitigen Festlegungsbereich (46, 246) aufweist, der ortsfest zum Gehäuse (12, 14a) festgelegt ist, **dadurch gekennzeichnet, dass**
     - zumindest ein Teilabschnitt des Ventiltellers (44; 144; 244; 386), der mit Flüssigkeit in der Druckkammer in Kontakt ist, zur Vermeidung von Verunreinigungen in der auszutragenden Flüssigkeit aus einem Kunststoff besteht, der unter Verwendung von Metallocenen als Katalysatoren hergestellt werden kann und es sich bei dem Kunststoff um ein Cyclo-Olefin-Copolymer oder um eine Kombination aus einem Cyclo-Olefin-Copolymer und einem Polypropylen handelt.

## Claims

1. Liquid dispenser (10) for discharging pharmaceutical liquids, having

     - a housing (12, 14a),
     - an outlet opening (16) and
     - an outlet valve (30) assigned to the outlet opening (16),

wherein
the outlet valve (30) comprises:

     - a valve opening (16; 382) and a valve pin (42; 370), the valve pin (42; 370) closing the valve opening (16; 382) in a closed position of the outlet valve and releasing the valve opening in an open position of the outlet valve,
     - a valve body (40; 140; 240; 380), which has either the valve pin (42) or the valve opening (382) and which is displaceable partially with respect to the housing (12, 14a) of the liquid dispenser (10) in order to thereby make it possible to transfer the outlet valve (30) between the closed position and the open position,
     - a pressure chamber (60), which is arranged upstream of the valve opening (16, 382) and is delimited to one side by a valve plate (44; 386) of the valve body (40; 140; 240; 380), the valve body (40; 140; 240; 380) being displaceable at least in portions by means of the application of pressure at the valve plate (44; 386) by liquid in the pressure chamber (60), such that the valve pin (42; 370) and the valve opening (16; 382) are thereby displaced against one another,
     - for the purpose of making it possible for the valve opening (382) or the valve pin (42) to be displaced with respect to the housing (12, 14a), the valve body (40; 140; 240; 380) has an intrinsically deformable configuration and has a fixing region (46, 246) on the outside which is fixed immovably in relation to the housing (12, 14a),
     **characterized in that**
     - at least one portion of the valve plate (44; 144; 244; 386) which is in contact with liquid in the pressure chamber consists of a valve plate material which is a plastic which can be produced using metallocenes as catalysts and the plastic is a cycloolefin copolymer or a combination of a cycloolefin copolymer and a polypropylene.

2. Liquid dispenser (10) according to Claim 1, **characterized in that**

   - the pressure chamber (60) has a pressure chamber volume $V_{DK}$ and the valve body (40; 140; 240; 380) has a pressure chamber surface area $A_{DK}$ facing toward the pressure chamber, and
   - the quotient of pressure chamber volume $V_{DK}$ divided by pressure chamber surface area $A_{DK}$ lies in the following interval:

$$0.1 \ mm \ \leq \ V_{DK} \ / \ A_{DK} \ \leq \ 2.4 \ mm$$

3. Liquid dispenser (10) according to Claim 2, **characterized in that** the quotient of pressure chamber volume $V_{DK}$ and pressure chamber surface area $A_{DK}$ lies in the following interval:

$$0.2 \ mm \ \leq \ V_{DK} \ / \ A_{DK} \ \leq \ 1.0 \ mm$$

4. Liquid dispenser (10) according to Claim 1, 2 or 3, **characterized in that** the pressure chamber volume $V_{DK}$ is between 20 mm$^3$ and 100 mm$^3$, preferably between 30 mm$^3$ and 50 mm$^3$.

5. Liquid dispenser (10) according to one of Claims 1 to 4, **characterized in that** the pressure chamber surface area of the valve body (40; 140; 240; 380) measures between 100 mm$^2$ and 300 mm$^2$.

6. Liquid dispenser (10) according to one of the preceding claims, **characterized in that** the valve opening (16) is provided immovably in relation to the housing (12, 14a) and the valve body (40; 140; 240) has the valve pin (42), the valve pin (42) being displaceable by at least partial displacement of the valve plate (44; 144; 244) with respect to the valve opening (16).

7. Liquid dispenser (10) according to Claim 6, **characterized in that** at least one portion of the valve plate (44; 144; 244) and the valve pin (42) are in the form of an integral component made of the plastic produced using metallocenes.

8. Liquid dispenser (10) according to one of the preceding claims, **characterized in that** the valve plate (144; 244) is provided with a circumferential deformation region (140a, 240a), in which the valve plate (44; 244) has a thinner form than in regions adjoining radially on the inside and outside.

9. Liquid dispenser (10) according to one of the preceding claims, **characterized in that** the outlet valve (30) has a valve spring (50), which subjects the valve pin (42) and the valve opening (16) to force in the direction of the closed position.

10. Liquid dispenser (10) according to one of the preceding claims, **characterized in that** the valve body (240) has a supporting body (247), which is displaceable together with the valve plate (244) and which consists of a plastic which is more rigid than the material of the valve plate.

11. Use of a plastic which has been produced using metallocenes as catalysts for a liquid dispenser (10) for discharging pharmaceutical liquids, wherein the liquid dispenser (10) comprises

   - a housing (12, 14a),
   - an outlet opening (16) and
   - an outlet valve (30) assigned to the outlet opening (16),

   wherein the outlet valve (30) comprises:

   - a valve opening (16; 382) and a valve pin (42; 370), the valve pin (42; 370) closing the valve opening (16; 382) in a closed position of the outlet valve and releasing the valve opening in an open position of the outlet valve,
   - a valve body (40; 140; 240; 380), which has either the valve pin (42) or the valve opening (382) and which is displaceable partially with respect to the housing (12, 14a) of the liquid dispenser (10) in order to thereby make it possible to transfer the outlet valve (30) between the closed position and the open position,
   - a pressure chamber (60), which is arranged upstream of the valve opening (16, 382) and is delimited to one side by a valve plate (44; 386) of the valve body (40; 140; 240; 380), the valve body (40; 140; 240; 380) being displaceable at least in portions by means of the application of pressure at the valve plate (44; 386) by liquid in the pressure chamber (60), such that the valve pin (42; 370) and the valve opening (16; 382) are thereby displaced against one another,
   - for the purpose of making it possible for the valve opening (382) or the valve pin (42) to be displaced with respect to the housing (12, 14a), the valve body (40; 140; 240; 380) has an intrinsically deformable configuration and has a fixing region (46, 246) on the outside which is fixed immovably in relation to the housing (12, 14a), **characterized in that**

- at least one portion of the valve plate (44; 144; 244; 386) which is in contact with liquid in the pressure chamber consists of a plastic which can be produced using metallocenes as catalysts for avoiding impurities in the liquid to be discharged and the plastic is a cycloolefin copolymer or a combination of a cycloolefin copolymer and a polypropylene.

**Revendications**

1. Distributeur de liquide (10) pour distribuer des liquides pharmaceutiques, comprenant

   - un boîtier (12, 14a),
   - une ouverture de sortie (16) et
   - une soupape de sortie (30) associée à l'ouverture de sortie (16),

   la soupape de sortie (30) comprenant :

   - une ouverture de soupape (16 ; 382) et une tige de soupape (42 ; 370), la tige de soupape (42 ; 370), dans une position de fermeture de la soupape de sortie, fermant l'ouverture de soupape (16 ; 382), et dans une position d'ouverture de la soupape de sortie, libérant l'ouverture de soupape,
   - un corps de soupape (40 ; 140 ; 240 ; 380), qui présente soit la tige de soupape (42) soit l'ouverture de soupape (382) et qui peut être déplacé en partie par rapport au boîtier (12, 14a) du distributeur de liquide (10) afin de permettre ainsi le transfert de la soupape de sortie (30) entre la position de fermeture et la position d'ouverture,
   - une chambre de pression (60) disposée en amont de l'ouverture de soupape (16, 382), qui est limitée vers un côté par une tête de soupape (44 ; 386) du corps de soupape (40 ; 140 ; 240 ; 380), le corps de soupape (40 ; 140 ; 240 ; 380) pouvant être déplacé au moins en partie au moyen d'une sollicitation par pression au niveau de la tête de soupape (44 ; 386) par du fluide dans la chambre de pression (60) de telle sorte que la tige de soupape (42 ; 370) et l'ouverture de soupape (16 ; 382) soient ainsi déplacées l'une par rapport à l'autre,
   - le corps de soupape (40 ; 140 ; 240 ; 380), afin de permettre le déplacement de l'ouverture de soupape (382) ou de la tige de soupape (42) par rapport au boîtier (12, 14a), étant réalisé de manière déformable en soi et présentant une région de fixation du côté extérieur (46, 246), qui est fixée en position fixe par rapport au boîtier (12, 14a), **caractérisé en ce que**
   - au moins une portion partielle de la tête de soupape (44 ; 144 ; 244 ; 386), qui est en contact avec le liquide dans la chambre de pression, se compose d'un matériau de tête de soupape qui est une matière plastique qui peut être fabriquée en utilisant des métallocènes en tant que catalyseurs, le plastique étant un copolymère de cyclo-oléfine ou étant une combinaison d'un copolymère de cyclo-oléfine et d'un polypropylène.

2. Distributeur de liquide (10) selon la revendication 1, **caractérisé en ce que**

   - la chambre de pression (60) présente un volume de chambre de pression $V_{DK}$ et le corps de soupape (40 ; 140 ; 240 ; 380) présentant une surface de chambre de pression $A_{DK}$ tournée vers la chambre de pression, et
   - le quotient du volume de chambre de pression $V_{DK}$ divisé par la surface de chambre de pression $A_{DK}$ est compris dans l'intervalle suivant :

$$0,1 \text{ mm} \leq V_{DK}/A_{DK} \leq 2,4 \text{ mm}.$$

3. Distributeur de liquide (10) selon la revendication 2, **caractérisé en ce que** le quotient du volume de chambre de pression $V_{DK}$ et de la surface de chambre de pression $A_{DK}$ est compris dans l'intervalle suivant :

$$0,2 \text{ mm} \leq V_{DK}/A_{DK} \leq 1,0 \text{ mm}.$$

4. Distributeur de liquide (10) selon la revendication 1, 2 ou 3, **caractérisé en ce que** le volume de chambre de pression $V_{DK}$ est compris entre 20 mm$^3$ et 100 mm$^3$, de préférence entre 30 mm$^3$ et 50 m$^3$.

5. Distributeur de liquide (10) selon l'une quelconque des revendications 1 à 4, **caractérisé en ce que** la surface de chambre de pression du corps de soupape (40 ; 140 ; 240 ; 380) est comprise entre 100 mm$^2$ et 300 mm$^2$.

6. Distributeur de liquide (10) selon l'une quelconque des revendications précédentes, **caractérisé en ce que**

l'ouverture de soupape (16) est prévue en position fixe par rapport au boîtier (12, 14a) et le corps de soupape (40 ; 140 ; 240) présente la tige de soupape (42), la tige de soupape (42) pouvant être déplacée par un déplacement au moins partiel de la tête de soupape (44 ; 144 ; 244) par rapport à l'ouverture de soupape (16).

7.  Distributeur de liquide (10) selon la revendication 6, **caractérisé en ce qu'**au moins une portion partielle de la tête de soupape (44 ; 144 ; 244) et de la tige de soupape (42) sont réalisées sous forme de composant d'une seule pièce en matière plastique qui est fabriquée en utilisant des métallocènes.

8.  Distributeur de liquide (10) selon l'une quelconque des revendications précédentes, **caractérisé en ce que** la tête de soupape (144 ; 244) est pourvue d'une région de déformation périphérique (140a, 240a) dans laquelle la tête de soupape (44 ; 244) est réalisée sous forme plus mince que dans les régions adjacentes radialement du côté interne et du côté externe.

9.  Distributeur de liquide (10) selon l'une quelconque des revendications précédentes, **caractérisé en ce que** la soupape de sortie (30) dispose d'un ressort de soupape (50) qui sollicite par force la tige de soupape (42) et l'ouverture de soupape (16) dans la direction de la position de fermeture.

10. Distributeur de liquide (10) selon l'une quelconque des revendications précédentes, **caractérisé en ce que** le corps de soupape (240) présente un corps de support (247) qui peut être déplacé conjointement avec la tête de soupape (244) et qui se compose d'un matériau plus rigide par rapport au matériau de la tête de soupape.

11. Utilisation d'une matière plastique qui a été fabriquée en utilisant des métallocènes en tant que catalyseurs pour un distributeur de liquide (10) pour distribuer des liquides pharmaceutiques, le distributeur de liquide (10) comprenant

    - un boîtier (12, 14a),
    - une ouverture de sortie (16) et
    - une soupape de sortie (30) associée à l'ouverture de sortie (16),

    la soupape de sortie (30) comprenant :

    - une ouverture de soupape (16 ; 382) et une tige de soupape (42 ; 370), la tige de soupape (42 ; 370), dans une position de fermeture de la soupape de sortie, fermant l'ouverture de soupape (16 ; 382) et dans une position d'ouverture de la soupape de sortie, libérant l'ouverture de soupape,
- un corps de soupape (40 ; 140 ; 240 ; 380), qui présente soit la tige de soupape (42) soit l'ouverture de soupape (382) et qui peut être déplacé en partie par rapport au boîtier (12, 14a) du distributeur de liquide (10), afin de permettre ainsi le transfert de la soupape de sortie (30) entre la position de fermeture et la position d'ouverture,
- une chambre de pression (60) montée en amont de l'ouverture de soupape (16, 382), qui est limitée vers un côté par une tête de soupape (44 ; 386) du corps de soupape (40 ; 140 ; 240 ; 380), le corps de soupape (40 ; 140 ; 240 ; 380) pouvant être déplacé au moins en partie au moyen d'une sollicitation en pression au niveau de la tête de soupape (44 ; 386) par du liquide dans la chambre de pression (60) de telle sorte que la tige de soupape (42 ; 370) et l'ouverture de soupape (16 ; 382) soient déplacées l'une par rapport à l'autre,
- le corps de soupape (40 ; 140 ; 240 ; 380), afin de permettre le déplacement de l'ouverture de soupape (382) ou de la tige de soupape (42) par rapport au boîtier (12, 14a), étant réalisé de manière déformable en soi et présentant une région de fixation du côté extérieur (46, 246) qui est fixée en position fixe par rapport au boîtier (12, 14a), **caractérisée en ce**
- **qu'**au moins une portion partielle de la tête de soupape (44 ; 144 ; 244 ; 386) qui est en contact avec le liquide dans la chambre de pression, pour éviter des impuretés dans le liquide à distribuer, se compose d'un plastique qui peut être fabriqué en utilisant des métallocènes en tant que catalyseurs et
la matière plastique étant un copolymère de cyclo-oléfine ou une combinaison d'un copolymère de cyclo-oléfine et d'un polypropylène.

*Fig. 1*

**Fig. 2**

*Fig. 3*

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102009006428 A1
- EP 0791397 B1
- WO 2012084557 A1
- DE 10201110 A1